Europäisches Patentamt

European Patent Office

Office européen des brevets

(11) Publication number: **0 414 932 A1**

# EUROPEAN PATENT APPLICATION

(21) Application number: 89115918.8

(51) Int. Cl.5: **A23G 3/30, A61K 9/68**

(22) Date of filing: 29.08.89

(43) Date of publication of application:
06.03.91 Bulletin 91/10

(84) Designated Contracting States:
AT CH DE ES FR GB LI

(71) Applicant: CSEMEGE EDESIPARI GYAR
Budafoki ut 187/189
Budapest, XI(HU)

(72) Inventor: Ignath, Ildik , Dr.
Piac tér 2
H-8000 Székesfehérvar(HU)
Inventor: Gogl, György
Bakator u. 13
H-1118 Budapest(HU)
Inventor: Gogl, Gabriella, Dr.
Bakator u. 13
H-1118 Budapest(HU)

(74) Representative: Hansen, Bernd, Dr.rer.nat. et al
Hoffmann, Eitle & Partner Patentanwälte
Arabellastrasse 4 Postfach 81 04 20
W-8000 München 81(DE)

(54) Chewing gum composition.

(57) The present invention relates to a chewing gum composition which comprises 25 to 90 % by weight of known gum base,
0.01 to 0.2 % by weight of sodiummonofluoro phosphate,
0.5 to 40 % by weight of calcium carbonate of a particle size of 1 to 80 $\mu$m,
0.05 to 2 % by weight of sodium benzoate,
0.2 to 40 % by weight of artificial sweetener, preferably sodium cyclamate, sodium saccharine, xyilite, sorbitol,
2 to 25 % by weight of softening agent, preferably glycerol, and
1 to 10 % by weight of perfume and/or flavourant, preferably a natural flavourant.
The chewing gum composition exerts its anticariogenic effect both locally and enterally, simultaneously assures a preferable condition for fluorine ions to build into the teeth by mechanical and ohemical effect, thus less fluorine compound is needed and the building-in of fluorine ions is accelerated,

EP 0 414 932 A1

## CHEWING GUM COMPOSITION

The present invention relates to a chewing gum composition comprising a fluorine compound, mechanical polishing agent, disinfectant and artificial sweetening agent together with other excipients in a chewing gum base as carrier. The chewing gum of the present invention is especially suitable for prevention of formation of caries or decreasing of the occurence of caries and other diseases of parodontium and assuring the hygiene of mouth.

It is a well-known fact that caries is a widespread disease and it is especially dangerous when children are attacked. The epidemic examinations in Hungary show that the frequency of the appearance of this disease in children and teenagers doubles in every ten years. As the destruction of teeth caused by the pathological process cannot be regenerated by the organism, the defence against of this disease has high significance.

The only possibility of defense is prevention, however, a really effective method of prevention is not known yet.

According to the men skilled in the art the three main principles which prevention is based on are the use of fluorides, the change of consuming habits and sufficient, regular clean of teeth.

The reason of caries is that the hydroxylapatite crystals forming about 95 % of the inorganic part of dental enamel "dissolve out" from the dental enamel. One of the methods of defense against caries is the use of fluorides. The basis of anticariogenic effect of fluorides is that they can build into the dental enamel before or after the damage of teeth and they cause such preferable changes which increase the capability of resistance.

When fluorides are administered, the following chemical reaction is aimed to occure on the damaged surface of teeth:

$$[Ca_3(PO_4)_2]_3 \, Ca(OH)_2 \, + \, 2 \, F^- \rightleftharpoons [Ca_3(PO_4)_2]_3 \, CaF_2 \, + \, 2 \, OH^-$$

i.e. the hydroxylapatite crystals transforme into stable, resistable fluorapatite crystals.

As to the use of fluorides, the addition of fluorides to drinking water and salt is known. However, the amount of fluorides administered cannot be controlled by this method, children who are most exposed to the danger of caries can consume less drinking water or salt which fluoride content would be sufficient for preventing the disease and the collective use of fluorides is not solved due to organisation technical problems.

The administration of tablets comprising fluoride to children attending infant's nurseries or kinder-gartens, is one of the best methods, however, only about 20 to 30 % of children being 2 to 3 years old can be treated in such way. On the other hand, the tablet has to be added each day and the daily fluorine dosage cannot be administered when the child is missing from the nursery.

By the administration of fluorine compound with drinking water, salt and tablet the fluorine compound may exert its activity only enterally by adsorption from the stomach. However, when it is enterally used, it has to be used in relatively high amounts and it has an action not only to teeth but to the whole organism, and non-desired side-effects can occure. Thus nowdays the enteral administration as the only way of administration of fluorine compounds is not suggested.

It is also known that fluorides can locally be administered in the form of gels, teeth pastes, by rinsing or painting. However, these forms of administration have not became wide-spread and their spreading is unsolved.

Useful defense can be the influence of the surroundings of teeth. Thus the unfavourable conditions for the development of caries can be influenced in mouth.

It can be established that fluorine can be administered to the organism locally or enterally, however, the most preferable way of prevention of caries is when it is used both locally and enterally as thus much less amount of fluorine compound has to be added enterally to achieve the same effect. The most preferable effect could be obtained when in addition to the local and enteral administration of fluorine compound, the conditions of mouth would also be influenced in such a way which would facilitate the building of fluorine ions into teeth.

It can be obtained, when the fluorine compound is added in the form of a chewing gum, as in this way the fluorine compound can act both orally and enterally and the gum base cleans the teeth, thus facilitates the contact of fluorine ions with the damaged tooth surface. On the other hand, children are pleased to chew a chewing gum, thus the children need not to be forced to have their daily fluorine dose.

According to Roumenian patent specification No. 59,633, US patent specification No. 4,265,877 and French patent specification No. 2,420,970 fluorine ion is administered in the form of chewing gum. In the Roumenian specification calcium fluoride is mixed to the chewing gum base together with vitamins, artificial sweeteners and disodium phosphate. In order to achieve the anticariogenic effect, free fluorine ions are needed. However, calcium fluoride is a stable compound, poorly dissolves in water, thus local effect cannot be achieved in mouth and its enteral adsorption is also poor.

In the French specification sodium fluoride and xilite together with sorbitol are used as fluorine compound and artificial sweeteners, respectively.

According to literature sodium fluoride is one of the most preferred compounds for preventing caries and it can be used in the form of tablet or painting lotion when dissolved in water. However, the toxicity of sodium fluoride is relatively high, much higher than that of e.g. sodium monofluorophosphate.

According to the above US patent specification sodium fluoride and powdered oyster-shell are incorporated into chewing gum base. The 97 % of the oyster-shell is calcium carbonate of organic origin and sodium fluoride reacts with calcium carabonate in the course of chewing and stable, poorly water-soluble thus difficultly absorbable calcium fluoride is formed. According to the specification the oyster-shell comprises 3 % of trace elements (e.g. magnesium, aluminium, etc.) inhibiting this reaction. With the aid of use the oyster-shell only the local effect of fluoride is aimed to be enhanced by inhibiting the formation of calcium fluoride. However, the hydroxylapatite/fluorapatite conversion occuring on the surface of teeth is influenced by the increase of the amount of fluorine, and therefore they use it in an amount being greater with an order of magnitude than in the above-cited French specification. Such high amount of sodium fluoride is toxic and it can be used under medical supervision.

However, useful defense cannot be obtained by the simple administration of fluorine, as the millieau of mouth highly influences the equilibrium between hydroxylapatite/fluorapatite formation. The actual free fluorine ion content is significantly influenced by the pH as in highly acidic medium the formation of free fluorine ions is supressed thus the equilibrium is shifted into the direction of formation of hydroxylapatite instead of fluorapatite.

According to the known solutions the suitably high free fluorine ion concentration is achieved by administering calcium- or sodium fluoride. Calcium fluoride poorly gives free fluorine ions as it is poorly soluble in water, while sodium fluoride is enterally toxic in the amount which is sufficient for obtaining.the desired local result. Moreover, the known chewing gum compositions are not suitable for chemically influencing the conditions in mouth, they have only a mechanical effect, thus do not accelerate the bulding of fluorine ions into teeth.

When working out the present invention our aim was to prepare a chewing gum comprising a fluorine compound which exerts its effect both locally and enterally.

Our further aim was to prepare a teeth protecting chewing gum in which the fluorine compound can be used in significantly smaller amount than in the known compositions for achieving the free fluorine ion concentration in mouth being sufficient to obtain the desired local effect, thus the toxic side-effect can be eliminated.

Our further aim was to work out a composition which has a preferable chemical influence to the conditions of oral cavity and thus facilitates and accelerates the building of fluorine ions into the hydroxylapatite crystals of teeth.

Surprisingly we have found that the above aims can be achieved by using a chewing gum comprising 25 to 90 % by weight of known gum base,
0.01 to 0.2 % by weight of sodiummonofluoro phosphate,
0.5 to 40 % by weight of calcium carbonate of a particle size of 1 to 80 $\mu$m,
0.05 to 2 % by weight of sodium benzoate,
0.2 to 40 % by weight of artificial sweetener, preferably sodium cyclamate, sodium saccha rine, xyilite, sorbitol,
2 to 25 % by weight of softening agent, preferably glycerol, and
1 to 10 % by weight of perfume and/or flavourant, preferably a natural flavourant.

The above aims can be achieved by the complex effect of sodiummonofluoro phosphate, calcium carbonate and sodium benzoate The local and enteral effect of fluorine ions are assured by sodiummmonofluorophosphate, the favourable conditions for exerting the effect of fluorine ion in oral cavity is assured by the chemical and antibacterial action of sodiummonof luoro phosphate and sodium benzoate and by the mechanical action of the gum base and calcium carbonate

Sodiummonofluoro phosphate exerts its effect on the one hand enterally, on the other hand locally, moreover acts as a catalyst for fluorineapatite formation and inhibits the unfavourable enzymatic decomposition processes in mouth

This compound is able not only to give free fluorine ions, but also enhances the ratio of the crystallization process of hydroxylapatite I.e the hydroxylapatite formation occures at the time and after the teeth are damaged. Hydroxylapatite is not formed directly but through e.g. an octacalcium phosphate (Ca$_8$-

$(HPO_4)_2(PO_4)_4)$ intermediate This intermediate is more water soluble compound, and if the transformation of this intermediate into hydroxylapatite is not complete, the possibility of caries is enhanced. Sodiummonofluoro phosphate increases the ratio of this transformation process even at low concentrations.

This fluorine compound simultaneously inhibits the emolase enzyme in glycolisis, thus forms a bar against glycolisis and against the formation of enolic pyruvic acid and pyruvic acid.

The optimal daily dose of fluorine is 1 to 2 mg which is assured by chewing 2 to 4 pieces of a chewing gum of 3 g weight. Slight toxic side effect would occure when 240 pieces of chewing gum would have been be chewed, thus the possibility of toxic side effect is practically zero

Thus the incorporation of fluorine into the teeth is significantly better, quicker and more effective than in the case of the known chewing gum compositions comprising a fluorine compound, and the amount of fluorine compound needed for achieving the anticariogenic effect is much less than in the known solutions, toxic side-effects cannot occure.

The chewing gum composition of the present invention comprises sodium benzoate. Sodium benzoate on the one hand assures the optimal pH (pH = 7 to 8.8) in mouth for forming the suitable fluorine ion concentration from sodiummonofluoro phosphate, on the other hand inhibits the development and multiplication of acid-tolerable acidophylic and acidogenic microorganisms (Streptococci, Lactobacilli, special yeast bacteria, etc.) responsible for caries

As it is known, monosaccharide is formed in the microflora of mouth from carbohydrates upon the activity of enzymes. When carbohydrates are decomposed, several non- volatile acids are formed which turn the pH value of the microflora of mouth into acidic. Sodium benzoate inhibits this acidification process by its disinfectant character and retains the pH corresponding to the microflora of oral cavity. As sodium benzoate exerts its effect only in acidic medium, it does not influence the microflora of the healthy oral cavity,

The use of sodium benzoate enables to keep the pH value of 7 to 8.8 in the oral flora and in the saburra and thus the substitution of hydroxyl ion with fluorine ion can be achieved with a smaller concentration of fluorine compound.

Sodium benzoate also serves as preserving agent for perfume and/or flavouring agents.

Calcium carbonate of a particle size of 1 to 80 $\mu$ serves as mechanical polishing agent. Calcium carbonate of the above-defined particle size together with the gum base has a mechanical effect, i.e it removes the acidic saburra from the surface of teeth, thus assures that the formed free fluorine ions can contact the damaged surface of teeth. This agent does not damage the dental enamel, however being a mechanical polishing agent, it is suitable for removing of saburra during chewing.

As carrier commercially available or any other known chewing gum base can be used. The chewing of chewing gum requires strong moving of mouth, due to chewing a pressing effect occures, opening powers occure on the surface of teeth which are very preferable from the point of view of building in of fluorine.

The chewing gum wear the surface of teeth flat during chewing, thus those tooth surfaces are eliminated which are most exposed to caries which is preferable from the point of view of resistance of teeth and parodontium. Due to its sialagogue effect it has a role in removing and diluting the saburra adhered to teeth.

The present composition comprises an artificial sweetening agent, e.g, sodium cyclamate, xylite instead of sugar, as this agent is not cariogenic. By substituting sugar with an artificial sweetener it can be achieved that fluorine can exert its effect in a carbohydrate-free medium under optimal conditions,

As perfume and flavouring agents, the composition of the present invention may comprise peppermint, penny-royal, cinnamon oil, lemon oil, the mixture thereof or synthetic perfume and flavouring agents.

The chewing gum composition of the present invention is prepared by homogenizing the powdered polishing agent, the disinfectant, the artificial sweetener and the fluorine compound. The chewing gum base is used in solid form which is heated to a temperature of 30 to 70 °C in order to facilitate mixing. Then the homogenized mixture together or without softener (e.g. glycerol oil) is gradually added to the chewing gum base at the above temperature and the mixture is homogenized. Then the mixture is gradually cooled to room temperature and the perfume and flavouring agents are mixed therein. Finally the flexibility of the product is adjusted by adding a softener.

The invention is illustrated by the following, non-limiting examples. As sodium monofluorophosphate Phoskadent Na 211[R] is used,

| Example 1 | |
|---|---|
| Sodium monofluorophosphate | 0.01 % by weight |
| Calcium carbonate (1 to 80 $\mu$) | 0.50 % by weight |
| Sodium cyclamate | 0.03 % by weight |
| Sodium benzoate | 0.05 % by weight |
| Gum base (SP-8091) | 97.91 % by weight |
| Glycerol | 0.5 % by weight |
| Peppermint | 1.0 % by weight |

| Example 2 | |
|---|---|
| Sodium monofluorophosphate | 0.12 % by weight |
| Calcium carbonate (1 to 80 $\mu$) | 38.00 % by weight |
| Sodium cyclamate | 3.00 % by weight |
| Sodium benzoate | 2.00 % by weight |
| Gum base (SP-8091) | 40.88 % by weight |
| Glycerol | 10.0 % by weight |
| Aroma substance SZ 120586 | 6.0 % by weight |

| Example 3 | |
|---|---|
| Sodium monofluorophosphate | 0.01 % by weight |
| Calcium carbonate (1 to 80 $\mu$) | 7.00 % by weight |
| Sodium cyclamate | 0.60 % by weight |
| Sodium benzoate | 1.00 % by weight |
| Gum base (SP-8091) | 86.10 % by weight |
| Glycerol | 3.00 % by weight |
| Aroma substance SZ 120586-2 | 2.00 % by weight |

| Example 4 | |
|---|---|
| Sodium monofluorophosphate | 0.11 % by weight |
| Calcium carbonate (1 to 80 $\mu$) | 9.00 % by weight |
| Sodium cyclamate | 1.00 % by weight |
| Sodium benzoate | 2.00 % by weight |
| Gum base (SP-8091) | 79.40 % by weight |
| Glycerol | 4.19 % by weight |
| Aroma substance SZ 110586-3 | 4.30 % by weight |

Test example 1

The effectiveness of the chewing gum composition was compared to that of the chewing gum compositions according to French patent specification No. 2,420,970 and US patent specification No 4,265,877.

The composition of Example 1 and the composition of Example 1 of French patent specification were compared.

The test was carried out on 16 children of 7 to 8 years age. A contact foil with a circled hole of determined area in the middle was sticked onto the labial surface of the incisor. The tooth surface appearing through the hole was examined.

The patients were divided into two groups. The patients in first group received two chewing gums prepared according to the French patent specification No. 4,265,877 (group 1), while the second group received two chewing gums prepared according to the present invention and they chewed it for 15 minutes. Then biopsy was carried out on the surface of teeth, and the fluorine ion concentration in the surface dental enamel was measured by the method described in Mikrobestimmung von Fluorid in Körperflüssigkeiten mit der kombinierten ionenselektiven Elektrode im hangenden, Tropfen, A, Végh, Budapest and Deutsche Zahnarztliche 38. Jahrgang, 1983.

It was found that the fluorine ion concentration of teeth of patients administered with the chewing gum according to the present invention was higher with an average of 38 90 than in the teeth of patients treated with the chewing gum according to the French patent specification.

Thus if the of effectivity of the chewing gum according to the French patent specification is considered as 100 %, then the effectivity of the chewing gum of the present invention is 150 %.

Test example 2

The test according to test example 1 was repeated, but the patients had rinsed their mouth with 10 % by weight of saccharose solution for 5 minutes, then 15 minutes after rinsing, they got the gums to chew. The patients in third group and the fourth group got 2 chewing gums each, prepared according to the present invention (group 4) and to the French patent specification No 4,265,877 (group 3), respectively, and they chewed it for 15 minutes Then biopsy was carried out on the surface of teeth, and the fluorine ion concentration in the surface dental enamel was measured.

The fluorine concentration in teeth belonging to group 3 was less with 42 % than in group 1.

If the fluorine concentration is considered as 100 % in group 1, the fluorine concentration in group 4 is 127 %. Thus in the second test the chewing gum of the present invention was 2,18 times more effective than the chewing gum of French patent specification No 4,265,867.

Test example 3

The comparative test was also made by using the chewing gum according to US patent specification No. 4,265,877 as control. The composition of the control chewing gum was as follows:

| Compound | mg | % by weight |
|---|---|---|
| sodium fluoride | 2.34 | 0.078 |
| calcium carbonate | 103.92 | 3.464 |
| sodium cyclamate | 180.00 | 6.000 |
| gum base | 2473.74 | 82.000 |
| softener | 120.00 | 4.000 |
| flavourant | 120.00 | 4.000 |
| | 3000.00 mg | 100.000 % |

The same test was carried out as in test example 2and it was found that the fluorine ion concentration of teeth of patients treated with the control chewing gum was less with 49 % compared to the patients treated with the chewing gum according to the present invention.

Thus the chewing gum of the present invention is two times more effective than the control one.

## Claims

1. Chewing gum composition which comprises 25 to 90 % by weight of known gum base,
0.01 to 0.2 % by weight of sodiummonofluoro phosphate,
0.5 to 40 % by weight of calcium carbonate of a particle size of 1 to 80 $\mu$m,

0.05 to 2 % by weight of sodium benzoate,
0.2 to 40 % by weight of artificial sweetener, preferably sodium cyclamate, sodium saccharine, xyilite, sorbitol,
2 to 25 % by weight of softening agent, preferably glycerol, and
1 to 10 % by weight of perfume and/or flavourant, preferpreferably a natural flavourant.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 206 991 (WARNER LAMBERT CO.) * Page 4, lines 14-17; page 5, lines 10-12; page 5, lines 28-35; page 8, lines 23-30; example 2 * | 1 | A 23 G 3/30 A 61 K 9/68 |
| Y | WO-A-8 800 463 (ORAL RESEARCH LABORATORIES) * Page 5, line 21 - page 8, line 1; page 8, lines 11-26; page 9, lines 16-22; page 11, line 12 - page 12, line 13; page 26, lines 16-18; example 5 * | 1 | |
| A | EP-A-0 263 224 (WARNER LAMBERT CO.) * Page 4, lines 41-46; claims 1,3,12,17 * | 1 | |
| A | NL-A-6 614 699 (COLGATE PALMOLIVE CO.) * Page 3, line 35 - page 4, line 14 * | 1 | |
| A | FR-A-2 345 938 (P. CHOAY) * Page 2, lines 5-9 * | 1 | TECHNICAL FIELDS SEARCHED (Int. Cl.5) A 23 G A 61 K |

The present search report has been drawn up for all claims

| Place of search | Date of completion of the search | Examiner |
|---|---|---|
| THE HAGUE | 13-10-1989 | LEPRETRE F.G.M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)